# EUROPEAN PATENT APPLICATION

(11) **EP 4 666 972 A1**
(43) Date of publication of application: **24.12.2025**
(21) Application number: 24919388.9
(22) Date of filing: 26.01.2024
(51) Int. Cl.: A61B 18/12, A61B 18/14

(54) **ABLATION CATHETER, ABLATION SYSTEM, AND ABLATION METHOD**

(71) Applicant: SUZHOU SINUS MEDICAL TECHNOLOGIES CO., LTD, Suzhou , Jiangsu 215125 (CN)
(72) Inventor: DONG, Yuanbo, Suzhou, Jiangsu 215125 (CN); ZHANG, Mingfang, Suzhou, Jiangsu 215125 (CN)
(74) Representative: Meissner Bolte Partnerschaft mbB
(86) International application number: PCT/CN2024/074325
(87) International publication number: WO 2025/156290

(57) **Abstract**

The present invention relates to the technical field of medical equipment, and provides an ablation catheter, an ablation system comprising the ablation catheter, and an ablation method. The ablation catheter comprises an introducer portion, an ablation body, a catheter body and a control handle which are connected in sequence; the introducer portion includes a screw-in member capable of being rotated into and fixed to the target tissue under external force; the ablation body comprises an ablation electrode, a connecting member and a temperature sensor, and the catheter body comprises a non-ablation electrode and an infusion channel. The non-ablation electrode is spaced from the ablation electrode, and does not contact with the target tissue during ablation. By using the ablation catheter and ablation system of the present invention, it is possible to screw into a thickened interventricular septum for ablation with better control over the speed of screwing in. This also allows timely cessation or depth adjustment before myocardial penetration, thus preventing myocardial perforation, and damage to myocardial tissue is reduced due to temperature detection and perfusion functions.

## Description

### Technical Field

The present invention relates to the technical field of medical equipment, and particularly to an ablation catheter, an ablation system and an ablation method.

### Background

Hypertrophic cardiomyopathy (HCM) is a myocardial disease characterized primarily by asymmetric hypertrophy of the heart. Cardiac thickening of a typical type usually occurs in the left ventricle, particularly in the interventricular septum, while other rare types involve apical hypertrophy, uniform hypertrophy, and left ventricular anterior wall hypertrophy. It is a genetic myocardial disease caused by mutations in genes encoding sarcomere/sarcomere-associated proteins. Ultrasound typically finds thickening of the left ventricular wall, often defined as ≥15 mm thickness of the interventricular septum or left ventricular wall measured by 2D echocardiography, or ≥13 mm thickness in those with a clear family history. This condition typically does not involve left ventricular chamber dilation, and a patient with severe interventricular septal hypertrophy may lead to obstruction of the left ventricular outflow tract, causing hemodynamic disturbances.

Current treatment options for hypertrophic cardiomyopathy include pharmacological, surgical interventions, etc. For refractory HCM patients, septal reduction therapy is the ultimate treatment strategy, but its application is limited due to surgical trauma and technical demands. Intracoronary chemical ablation serves as a primary minimally invasive alternative of septal reduction therapy for some patients, yet its efficacy heavily depends on the anatomy of the interventricular septal artery. Recently, endocardial and intramyocardial radiofrequency ablations have been introduced for alleviating left ventricular outflow tract (LVOT) obstruction in HCM patients.

The prior art has disclosed an ablation solution for treating hypertrophic obstructive cardiomyopathy, primarily targeting septal thickness within the range of 10-20 mm. However, clinically, a patient may present with a thicker interventricular septum up to 30 mm, where the ablation catheter in the prior art may fail to ensure the effect of comprehensive ablation.

In addition, current treatment protocols rely on expensive 3D imaging/CT imaging devices for experimental detection of the interventricular septum. This is disadvantageous for widespread use of a septal pulse ablation catheter, as smaller medical facilities are unable to guarantee full device availability. Consequently, in a case of the absence of such devices, it remains one of the technical challenges in the prior art how to enable clear understanding of septal conditions and precise position of the septal pulse ablation catheter for an operator.

### Summary of Invention

To address the above technical issue in the prior art, the present invention aims to provide an ablation catheter for treating hypertrophic cardiomyopathy, an ablation system comprising said ablation catheter, and an ablation method, which is capable of using a simple imaging device to determine the position of the ablation catheter and achieve improved ablation effectiveness even for a thicker interventricular septum. Furthermore, the ablation catheter in the present invention minimizes damage to normal myocardial tissue upon catheter removal.

To achieve the above objective, the technical solution of the present invention is as follows:
In one aspect of the present invention, there is provided an ablation catheter comprising an introducer portion, an ablation body, a catheter body, and a control handle which are connected in sequence;
wherein,
the introducer portion comprises a screw-in member capable of being rotated into and fixed to the target tissue under external force;
the ablation body comprises an ablation electrode, a connecting member and a temperature sensor; the ablation electrode has a first end joined with the screw-in member, a first cavity arranged opposite the first end, a second cavity formed by extending from the first cavity towards the first end and a perfusion hole extending through the circumferential surface of the ablation electrode; inside the first cavity is provided the connecting member, and one end of the connecting member, which is near the catheter body, extends beyond the first cavity and joins with the catheter body; the connecting member has a first channel running through axially, the first channel communicates with the second cavity, the perfusion hole and an infusion channel in the catheter body; the connecting member and an inner wall of the first cavity form a number of second channels, with the temperature sensor located in one of the second channels;
the catheter body comprises a non-ablation electrode and the infusion channel, with the non-ablation electrodes spaced from the ablation electrode, and the non-ablation electrode is not in contact with the target tissue during ablation.

Preferably, position information for the ablation electrode is provided by the impedance difference between the ablation electrode and the non-ablation electrode.

Preferably, the screw-in member is in a three-dimensional helical line or screw shape, with the end away from the ablation body as a tip.

More preferably, the pitch of the screw-in member gradually decreases from a distal end to a proximal end, and the pitch is 0.25-1.5 times the diameter of the screw-in member.

More preferably, the diameter of the screw-in member gradually decreases from a proximal end to a distal end.

Preferably, the first end of the ablation electrode has a third cavity, into which the screw-in member is connected.

More preferably, the screw-in member is fixedly connected into the third cavity, and the length of the screw-in member is greater than the length of the third cavity in the axial direction.

More preferably, the screw-in member is telescopically connected into the third cavity, and the length of the screw-in member is less than or equal to the length of the third cavity in the axial direction.

Preferably, the introducer portion further includes an insert, the length of which is less than or equal to the length of the screw-in member, and the insert is sleeved inside the screw-in member and does not contact the screw-in member.

More preferably, the insert has a hollow structure and a through hole extending through the circumferential surface of the insert, and a proximal end of the insert is in communication with the second cavity.

Preferably, the perfusion hole is provided on one side near the first cavity of the second cavity.

Preferably, the perfusion hole is set in a plurality of groups, and the plurality of groups of perfusion holes are arranged spaced on the outer circumference surface of the second cavity along the axial direction of the ablation electrode.

More preferably, each group of the perfusion holes comprises a plurality of perfusion sub-holes which are spaced circumferentially around the ablation electrode.

More preferably, the projection of two adjacent perfusion holes overlaps partially on the plane perpendicular to the axial direction of the ablation electrode.

Preferably, the introducer portion conducts electricity and is insulated from the ablation electrode.

Preferably, the ablation body further comprises a positioning sensor configured to sense the position of the ablation catheter in the target tissue, and the temperature sensor and the positioning sensor are located in different second channels.

Preferably, the ablation catheter further comprises a guiding member comprising a connecting portion, a guiding portion, and a guiding hole extending through the connecting portion and the guiding portion; the connecting portion is joined with the ablation catheter, and the inner wall of the guiding portion is in a trumpet shape which tapers from one end away from the connecting portion to one end near the connecting portion; the guiding hole is in communication with a liner wire channel in the ablation catheter.

More preferably, the guiding member further comprises a gripping portion between the connecting portion and the guiding portion, and the guiding hole extends through the connecting portion, the gripping portion and the guiding portion.

According to another aspect of the present invention, there is provided an ablation system comprising the ablation catheter described above, an integrated ablation device, a perfusion device and an imaging device; the integrated ablation device is connected to the ablation catheter, the perfusion device and the imaging device respectively, and the perfusion device is joined with the ablation catheter; the integrated ablation device comprises an ablation generation module, a temperature detection module, an impedance detection module, an ECG signal detection module, an electrical stimulation/pacing signal module, a MCU central control module, an interactive control module and an ablation control switch;
wherein the ablation generation module is used to generate and deliver ablation energy to the ablation electrode;
the temperature detection module is used to receive a signal from the temperature sensor and feed back to the MCU central control module;
the impedance detection module is used to detect the impedance of the ablation electrode and/or the non-ablation electrode and feed back to the MCU central control module;
the ECG signal detection module is used to detect an electrocardiogram signal and feed back to the MCU central control module;
the electrical stimulation/pacing signal module is used to generate and send an electrical stimulation/pacing signal through the ablation electrode under the control of the MCU central control module;
the MCU central control module is in signal connection with the ablation generation module, the temperature detection module, the impedance detection module, the ECG signal detection module, the electrical stimulation/pacing signal module, the interactive control module, the ablation control switch, the ablation catheter, the perfusion device and the imaging device for receiving information from other modules for processing and providing feedback and/or display of processed information;
the interactive control module is used to display information and receive a control instruction from a user;
the ablation control switch is used to control application of ablation energy to the target tissue.

In yet another aspect of the present invention, there is provided an ablation system comprising at least two ablation catheters described above, an integrated ablation device, a perfusion device and an imaging device; the integrated ablation device is connected to the two ablation catheters, the perfusion device and the imaging device respectively, and the perfusion device is connected to the two ablation catheters respectively; the integrated ablation device comprises an ablation generation module, a temperature detection module, an impedance detection module, an ECG signal detection module, an electrical stimulation/pacing signal module, a MCU central control module, an interactive control module, and an ablation control switch;
wherein the ablation generation module is used to generate and deliver ablation energy to each of the ablation electrodes;
the temperature detection module is used to receive a signal from each of the temperature sensors and feed back to the MCU central control module;
the impedance detection module is used to detect the impedance of each of the ablation electrodes and/or the non-ablation electrodes and feed back to the MCU central control module;
the ECG signal detection module is used to detect an electrocardiogram signal and feed back to the MCU central control module;
the electrical stimulation/pacing signal module is used to generate and send an electrical stimulation/pacing signal through each of the ablation electrodes under the control of the MCU central control module;
the MCU central control module is in signal connection with the ablation generation module, the temperature detection module, the impedance detection module, the ECG signal detection module, the electrical stimulation/pacing signal module, the interactive control module, the ablation control switch, the two ablation catheters, the perfusion device and the imaging device for receiving information from other modules for processing and providing feedback and/or display of processed information;
the interactive control module is used to display information and receive a control instruction from a user;
the ablation control switch is used to control application of ablation energy to the target tissue.

Preferably, the ablation system further includes a position detection module which receives a signal from a positioning sensor and feeds back to the MCU central control module.

In yet another aspect of the present invention, there is provided an ablation method using the ablation system comprising one ablation catheter as described above, the ablation method comprising the steps of:
S1: Subclavian vein/jugular vein puncture, sending the ablation catheter into the right ventricle via a delivery catheter;
S2: Under the guidance of the imaging device, adjusting the angle and position of the delivery catheter and the ablation catheter, and monitoring impedance and/or electrocardiogram waveforms in real-time, wherein the impedance is impedance between the ablation electrode and a negative plate on the patient's body surface, and/or impedance difference between the ablation electrode and the non-ablation electrode;
S3: After the delivery catheter reaches the designated position, screwing the introducer portion into the target tissue, determining, during the process, whether the introducer portion is screwed in and the depth of screwing in based on changes in impedance between the ablation electrode and the negative plate on the patient's body surface and/or changes in impedance difference between the ablation electrode and the non-ablation electrode, and activating the perfusion device once the ablation electrode reaches a target ablation area;
S4: Adjusting a parameter for ablation;
S5: After completing ablation, performing electrical stimulation to check if the ablation is sufficient;
S6: Unscrewing;
S7: Repeating S2-S6 if ablation of additional sites is required;
S8: After completing all operations, removing the catheter from the body.

Preferably, the ablation catheter includes a positioning sensor, and step S2 further comprises that the ablation catheter determines the position of the ablation electrode by the positioning sensor using magnetoelectric positioning.

Preferably, in step S1, femoral artery puncture is performed, sending the ablation catheter into the left ventricle via the delivery catheter.

Preferably, in step S3, an electrical signal is emitted by the ablation electrode, and whether the introducer portion is screwed in and the depth of screwing in are determined based on whether the electrical signal can be detected on the body surface and a change in the electrical signal.

Preferably, in step S5, the electrical stimulation check involves applying a progressively increasing pacing voltage electrical signal to the ablated tissue via the ablation electrode. When the applied pacing voltage electrical signal exceeds or equals a pacing voltage threshold, and an electrocardiogram captured by the electrical signal can be detected on the body surface, the completed ablation damage range is a desired ablation range corresponding to the pacing voltage threshold. The pacing voltage threshold represents a minimum voltage that, after completing the desired ablation damage range, can be transmitted to non-ablated tissue via ablated tissue, and the pacing voltage threshold is directly proportional to the desired ablation damage range.

The main beneficial effects achieved by the present invention are as follows:
1. For hospitals, existing determination of position by ablation catheters must be accompanied by a complete three-dimensional mapping system, which is prohibitively expensive and costs millions of dollars. Such a high cost is a significant barrier for ordinary hospitals, limiting the development of an ablation surgery. The ablation catheter of the present invention, however, can achieve screwing in and positional determination under the guidance of the simplest imaging device through coordination between the ablation electrode and the non-ablation electrode (e.g., detecting impedance difference between two electrodes, changes in electrocardiogram signals) and/or the positioning sensor, etc. This significantly reduces the basic requirements for conducting a surgery and lowers the learning difficulty for a doctor, thus expanding the scope of surgical benefits for wider adoption.
2. In prior arts, if the depth of a single screwing in of the introducer portion exceeds the depth itself, there is a high probability that part of myocardial tissue will be attached to the introducer portion in the removal process, causing damage to the interventricular septum. The present inventor aims to minimize such damage. In one embodiment of the present invention, the construction of the screw-in member is optimized by the inventor with a conical helical structure that facilitates easier screwing in and unscrewing. In another embodiment, the inventor optimizes the helical structure of the screw-in member by defining the relationship between pitch and diameter, thus ensuring stable screwing in and preventing tissue removal and excessive damage during unscrewing.

The use of the ablation catheter and ablation system in the present invention enables simpler and more effective treatment of hypertrophic cardiomyopathy, particularly achieving superior treatment outcomes for a patient with interventricular septum thickness exceeding 20 mm, while reducing damage to myocardial tissue. Moreover, the ablation catheter and ablation system in the present invention has a lower cost and a wider range of applicability.

### Description of Drawings

FIG.1 is a structural schematic diagram of an ablation catheter according to one embodiment of the present invention;
FIG.2 is a partial structural schematic diagram of an ablation catheter according to one embodiment of the present invention;
FIG.3 is a structural schematic diagram of a screw-in member 11;
FIG.4 is a structural schematic diagram of an insert according to one embodiment of the present invention;
FIG.5 is a structural schematic diagram of an insert according to one embodiment of the present invention;
FIG.6 is a structural schematic diagram of an ablation electrode according to one embodiment of the present invention;
FIG.7 is a structural schematic diagram of a perfusion hole;
FIG.8 is a structural schematic diagram of a connecting member according to one embodiment of the present invention;
FIG.9 is a structural schematic diagram of an ablation body according to one embodiment of the present invention;
FIG.10 is a structural schematic diagram of an ablation body according to one embodiment of the present invention;
FIG.11 is a structural schematic diagram of an ablation body and a catheter body according to one embodiment of the present invention;
FIG.12 is a structural schematic diagram of a guiding member according to one embodiment of the present invention;
FIG.13 is a structural schematic diagram of a guiding member according to one embodiment of the present invention;
FIG.14 is a partial schematic diagram of an ablation catheter according to one embodiment of the present invention;
FIG.15 is a schematic diagram showing changes in impedance of the ablation electrode when it is in different positions;
FIG.16 is a schematic diagram showing the determination of ablation range by an electrocardiogram signal;
FIG.17 is a schematic diagram of an ablation system according to one embodiment of the present invention;
FIG.18 is a schematic diagram of the use of an ablation system according to one embodiment of the present invention;
FIG.19 is a schematic diagram of an ablation system according to one embodiment of the present invention;
FIG.20 is a schematic diagram showing the positions of an ablation catheter in a heart;
FIG.21 shows an electrocardiogram in lead V1 when an ablation catheter is in different positions.

### Embodiments

### Definitions

Distal side: In this specification, when the term "distal side" is used to describe the apparatus in the present invention, it denotes the side that is relatively far from a user.

Proximal side: In this specification, when the term "proximal side" is used to describe the apparatus in the present invention, it denotes the side that is relatively close to a user.

Distal end: In this specification, when the term "distal end" is used to describe the system or apparatus in the present invention, it generally refers to the end that is relatively far from a user.

Proximal end: In this specification, when the term "proximal side" is used to describe the system or apparatus in the present invention, it generally refers to the end that is relatively close to a user.

Terms "mount", "join", "connect", "fix" and their derivatives are intended to have an inclusive meaning. For example, unless otherwise explicitly defined, they may include fixed connection, removable connection or integration; they may be mechanical connection or electrical connection; they may include direct joining and indirect joining through an intermediary medium; they may also refer to internal communication between two elements or their interaction. For an ordinary person skilled in the art, these terms may be understood according to the specific context in which they are used in the present invention.

Preferred embodiments of the present invention will now be described with reference to the accompanying drawings. It will be appreciated by those of skill in the art that the embodiments or examples described with reference to the accompanying drawings are merely illustrative of the best way of implementing the present invention, rather than limiting the scope of the present invention to these embodiments. Variations and modifications can be made based on the following embodiments in the present invention, and these variations and modifications are within the scope of the present invention.

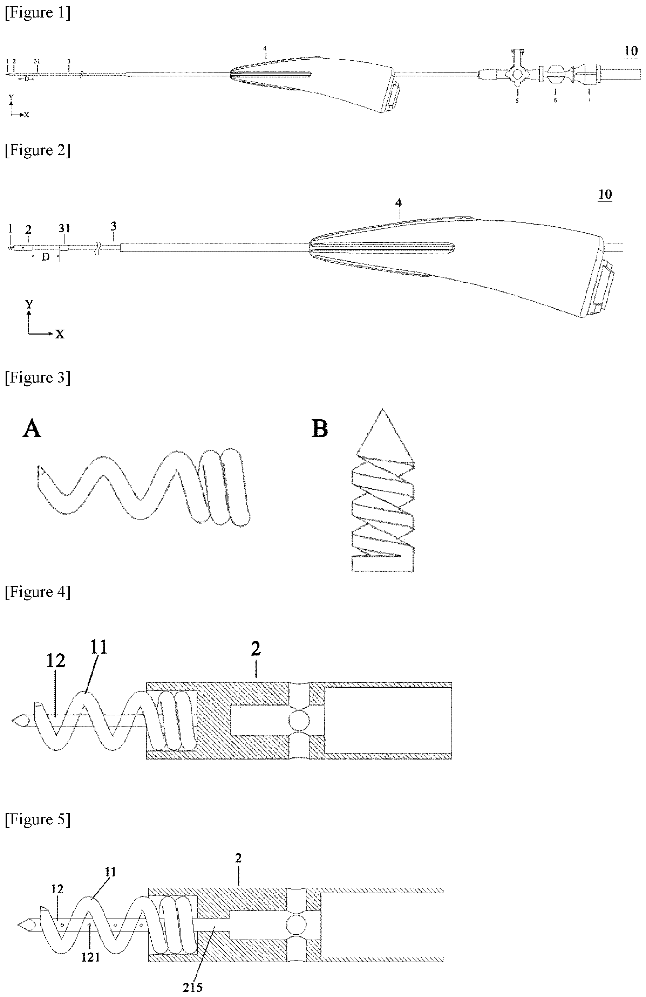

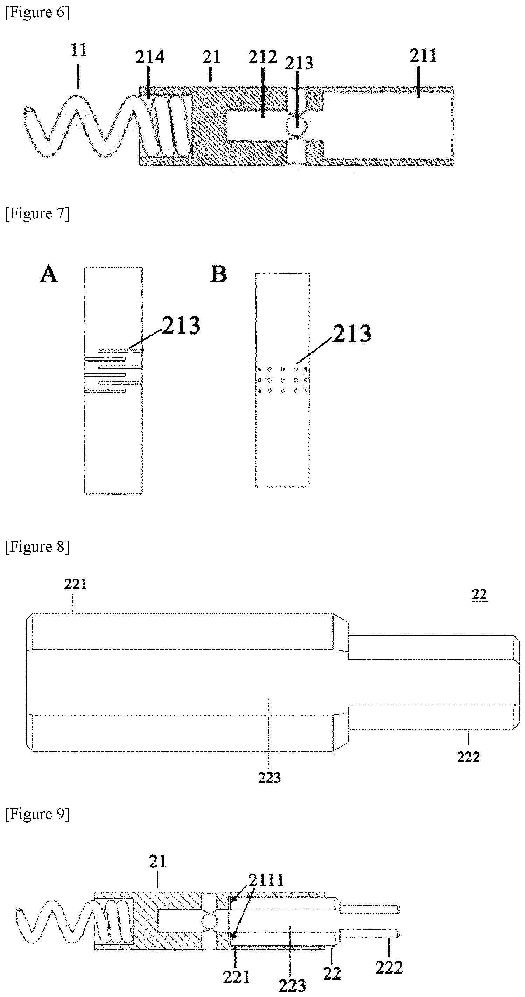

FIGS. 1 and 2 illustrate an ablation catheter 10 in one embodiment of the present invention, comprising an introducer portion 1, an ablation body 2, a catheter body 3, and a control handle 4 which are connected in sequence from the distal end to the proximal end. The catheter body 3 comprises a non-ablation electrode 31, which is spaced from an ablation electrode on the ablation body 2. During ablation, the non-ablation electrode 31 does not contact the target tissue. The introducer portion 1 includes a screw-in member 11 (FIG. 4) which can be rotated into and fixed to the target issue under external force, thereby fixing the ablation catheter 10 in the target ablation position and preventing position deviation of the ablation catheter 10 during the ablation of the target tissue, which may otherwise damage other non-target tissues or affect the ablation effectiveness. The ablation body 2 comprises an ablation electrode 21 configured as a shell, a connecting member 22, and a temperature sensor 23 (FIGS. 9-10). The ablation electrode 21 has a first end joined with the screw-in member 11, a first cavity 211 provided opposite the first end, a second cavity 212 formed by extending from the first cavity 211 towards the first end, and a perfusion hole 213 extending through the axial surface of the ablation electrode 21 (FIG. 6). The ablation electrode 21 is made of conductive material and is configured to contact the target tissue and emit ablation energy, such as radio frequency energy or pulse energy, to ablate the target tissue. The ablation electrode 21 is capable of extending into the target tissue with the introducer portion 1 or abutting against the surface of the target tissue for ablation. The connecting member 22 is used to connect the ablation body 2 and the catheter body 3, and the temperature sensor 23 is used to sense the temperature of the ablation electrode 21. Based on the measured temperature data, the power of the ablation electrode 21 can be adjusted in a timely manner to ensure that the temperature of the ablation site is stable without exceeding a threshold, thereby preventing thermal accumulation during the ablation of the target tissue, i.e., avoiding causing burns to surrounding tissue due to high electrode temperature and damage to the human body as a result. The type of the temperature sensor 23 is not particularly defined. In one embodiment of the present invention, the temperature sensor 23 is a thermocouple for sensing the temperature of the ablation catheter, and in a preferred embodiment of the present invention, the temperature sensor 23 is a K/T/J-type thermocouple. In this description of the present invention, the X-axis direction in FIG. 1 is the axial direction of the ablation catheter 10 in the present invention.

The introducer portion 1 includes a screw-in member 11 which is in a three-dimensional helical shape, as shown in FIG. 3A. The tip is the end of the screw-in member 11 away from the ablation body 2, and the catheter body 3 is configured to drive the screw-in member 11 to rotate under external force and fix into the target tissue. The tip is the end away from the catheter body 3 of the screw-in member 11, which allows easier penetration of the screw-in member 11 into the target tissue. Furthermore, as the screw-in member 11 is in a three-dimensional helical shape, it can abut with the surrounding tissue when it drills into the target tissue, preventing axial and radial movement of the ablation electrode 21 and ensuring reliable fixation of the screw-in member 11 within the target tissue. Upon completion of ablating one target tissue site, the screw-in member 11 can be unscrewed through the catheter body 3 to detach from the target tissue, and then reused to drill into the next target site for ablation until all target tissue sites are ablated.

Furthermore, as shown in FIG. 2, in some embodiments, the ablation body 2 is generally cylindrical. The screw-in member 11 may extend along a direction away from the ablation body 2 in the axial direction of the cylindrical ablation body 2 in a three-dimensional helical form, and as shown in FIG. 3, the end of the screw-in member 11 away from the ablation electrode 21 may be the tip. The screw-in member 11 can be rotated under the drive of the catheter body 3 and fixed into the target tissue. Optionally, the tip of the screw-in member 11 may be tapered, or may have other fairly sharp-pointed shapes obtained by grinding the end face of the free end of the screw-in member 11, such as a slice shape. In other words, the tip shape of the screw-in member 11 is not limited and aims to facilitate easier penetration into the target tissue.

In one embodiment of the present invention, the wire diameter of the screw-in member 11 is 0.1-0.4 mm, preferably 0.15-0.3 mm, with a diameter between 0.5-2 mm, and a number of helical turns between 2-10 turns. In one preferred embodiment, the length of the screw-in member 11 is 1-8 mm, more preferably 3 mm. In one preferred embodiment, the pitch of the screw-in member 11 is greater than or equal to 0.25 times the diameter of the screw-in member 11 and less than or equal to 1.5 times the diameter of the screw-in member 11, preferably 0.8 times the diameter of the screw-in member 11. In a case where the pitch of the screw-in member 11 is less than 0.25 times the diameter, it may be difficult to be screwed into myocardial tissue, which is disadvantageous for fixation, and would increase the removal of target tissue when unscrewing, causing excessive damage. Conversely, a large ratio between the pitch and diameter of the screw-in member 11 would lead to instability and shaking during the screw-in process when the material hardness of the screw-in member 11 remains unchanged. By optimizing the ratio between the pitch and diameter of the screw-in member 11, the present invention is able to conveniently screw the screw-in member 11 into the target tissue while reducing damage to the target tissue.

In one embodiment of the present invention, the screw-in member 11 is in a cylindrical helical line shape, and in another embodiment, the screw-in member 11 is in a conical helical line shape, where the diameter of the screw-in member 11 gradually decreases from the proximal end to the distal end. When the screw-in member 11 is in a conical helical shape, it is easier to be screwed into the target tissue and can reduce damage to the target tissue.

In other embodiments, the screw-in member 11 may also be screw-shaped, that is, the main body is cylindrical, with at least one sharp helical protrusion spiraling around the outer circumferential surface of the main body (FIG. 3B).

Additionally, in one embodiment of the present invention, the pitch of the screw-in member 11 varies, i.e., gradually decreasing from the distal end to the proximal end. Such arrangement facilitates easier screwing in of the distal end of the screw-in member 11 into the target tissue and provides a more secure fixation between the proximal end and the electrode.

It should be noted that during the treatment process, in some embodiments, a user can operate the catheter body 3 as required so as to adjust the drilling depth of the screw-in member 11 into the target tissue for better ablation treatment. In other embodiments, a user can also operate the control handle 4 exposed outside the patient's body to drive the catheter body 3 and the ablation body 2 which is connected to the catheter body 3, thereby driving the screw-in member 11 on the ablation body 2 to drill into or unscrew from the target tissue.

In one embodiment of the present invention, the screw-in member 11 is made of insulating material, or made of metal material with its outer surface made of insulating material. In this embodiment, the screw-in member 11 only serves to screw in and fix. In another embodiment of the present invention, the screw-in member 11 is a helical electrode head made of conductive material, including but not limited to platinum-iridium alloy, gold, nickel-cobalt-chromium-molybdenum alloy, stainless steel, titanium alloy and other metals. In this case, the screw-in member not only plays a role in screwing in and fixing, but also in ablating, mapping, pacing, etc. When the screw-in member 11 is made of conductive material, it may be conductive with the ablation electrode 21 and serve as part of the ablation electrode 21, or be insulated from the ablation electrode 21 and connected to the screw-in member 11 and the ablation electrode 21 respectively by separate conductor wires. The screw-in member 11 and the ablation electrode 21 may have opposite or same polarity.

The screw-in member 11 is fixed on the ablation electrode 21. When the ablation electrode 21 of the present invention has a third cavity 214 at its first end (as shown in FIG. 6), the screw-in member 11 is connected into the third cavity 214. In one embodiment of the present invention, the screw-in member 11 is fixedly connected into the third cavity 214, and the length of screw-in member 11 is greater than the length of the third cavity 214 in the axial direction, so that the tip of the screw-in member 11 is exposed outside the third cavity 214, facilitating screwing into the target tissue. Additionally, the screw-in member 11 may also be telescopically connected into the third cavity 214, with the length of the screw-in member 11 being less than or equal to the length of the third cavity 214 in the axial direction. That is, when the ablation catheter 10 is not used and/or the ablation catheter 10 enters into the human body, the screw-in member 11 is housed within the ablation body 2. Upon nearing the target tissue, the screw-in member 11 extends from the ablation body 2, thereby reducing inadvertent damage to other tissues by the head end of the ablation catheter 10 during surgery. Common methods in the art may be used to enable the screw-in member 11 to be telescopically connected into third cavity 214, such as the use of an electromagnetic spring, cooperation between a motor and a screw/gear, etc., so that the screw-in member 11 can extend or retract within the third cavity 214.

Further, as shown in FIGS. 4-5, the introducer portion 1 also comprises an insert 12 configured to be inserted into the target tissue and/or to ablate the tissue part with the screw-in member 11 screwing in. The screw-in member 11 sleeves around the periphery of the insert 12 without contacting the insert 12. In one embodiment of the present invention, the length of the screw-in member 11 exceeds that of the insert 12, such that when the screw-in member 11 is not screwed in, the insert 12 is surrounded within the screw-in member 11 and will not injure human tissues. Similar to the screw-in member 11, the insert 12 may be insulated or conductive. When the insert 12 is conductive, it may be conductive with the ablation electrode 21 and serve as part of the ablation electrode 21, or be insulated from the ablation electrode 21 and connected to the screw-in member 11 and the ablation electrode 21 respectively by separate conductor wires.

In one embodiment of the present invention, the insert 12 has a hollow structure and includes a through hole 121 extending through the circumferential surface of the insert 12 (FIG. 5). The proximal end of the insert 12 is in communication with the second cavity 212 via a channel 215, allowing fluid to flow out of the insert 12 from the through hole 121 via the second cavity 212. In another embodiment of the present invention, the insert 12 is telescopically arranged, that is, when the ablation catheter 10 is not used and/or the ablation catheter 10 enters into the human body, the insert 12 is housed within the ablation body 2. Upon nearing the target tissue, the insert 12 extends from the ablation body 2, thereby reducing inadvertent damage to other tissues by the head end of the ablation catheter 10 during surgery. Similar to the screw-in member 11, control over the telescoping of the insert 12 may be achieved using common methods in the art.

Referring to FIG. 6, the distal end of the ablation electrode 21 has a first end joined with the screw-in member 11. In this embodiment, the first end has a third cavity 214 extending towards the second cavity 212, wherein the screw-in member 11 is fixed within the third cavity 214. However, there are no particular limitations on the structure as long as the first end of the ablation electrode 21 can secure the screw-in member 11. For example, the first end can be a solid structure, with a portion of the screw-in member 11 buried in the first end for fixation, or the screw-in member 11 and the first end may be integrally formed. Connection between the screw-in member 11 and the ablation electrode 21 may be achieved using known methods in the art depending on practical application. For example, they may be fixedly connected by bonding, riveting, press-fitting, welding, etc., or removably connected through snap-fitting, threaded connection, etc.

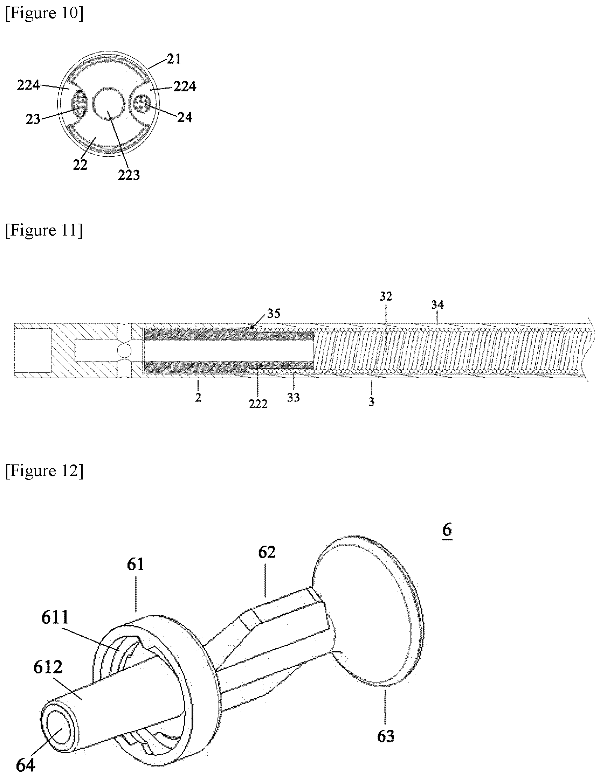

Near the proximal end of the ablation electrode 21 and opposite the first end, there is provided a first cavity 211 within which a connecting member 22 is disposed (as shown in FIG. 8). The second cavity 212, the perfusion hole 213 and the first channel 223 in the connecting member 22 are in communication with an infusion channel 32 in the catheter body 3 (as shown in FIG.11). Saline solution and other fluids delivered via the infusion channel 32 is delivered to the second cavity 212 through the first channel 223, and then perfused into the ablation electrode 21 and the target tissue through the perfusion hole 213, reducing the temperature of the ablation electrode 21 and the target tissue.

The ablation electrode 21 may be fabricated from any material commonly used in the art for electrode manufacturing without specific limitation, such as gold, copper, titanium alloy, platinum, stainless steel, etc. In one embodiment, the ablation electrode 21 is coated with an arc preventive adhesive to reduce arc generation during ablation, thereby enhancing safety.

Additionally, in other embodiments of the present invention, the ablation body 2 includes an insulating housing and at least two ablation electrodes spaced along the axial direction of the insulating housing. In this embodiment, the structure of the insulating housing may be identical to that of the ablation electrode 21 described above in the present invention, with the only distinction of being non-conductive; therefore, no more details will be described here. The at least two ablation electrodes may be ring electrodes fitted on the insulating housing, with each joined with the control handle via a conductor wire. A number of perfusion holes are provided on the circumferential surface of the ring electrodes. Reasonable electrode spacing and an ablation parameter are set between the at least two ablation electrodes, which, upon completion of ablation, can create a continuous ablation area. In one preferred embodiment, the polarity of the two ablation electrodes is opposite. In one preferred embodiment, the spacing between the two ablation electrodes is in a range of 1-6 mm. In one preferred embodiment, the voltage applied to each ablation electrode ranges from 800-3600V.

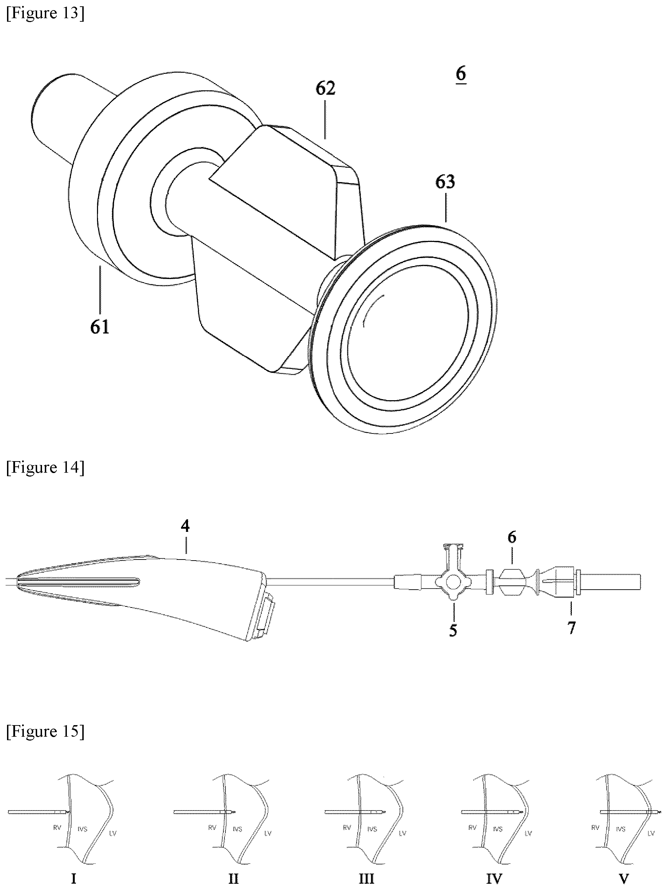

In the present invention, the ablation electrode 21 not only serves to ablate target tissue, but also enables determination of the position of the ablation catheter 10 before ablation begins. Due to varying electric conductivities in blood and muscles, as well as in different locations of the same muscle tissue, the impedance of the ablation electrode 21 in different locations also varies (forming a loop by providing a negative plate on the body surface). As shown in FIG. 15, when the ablation electrode 21 only contacts blood without entering the interventricular septum (I), its impedance is relatively low. However, as the ablation electrode 21 enters the interventricular septum (II) towards the central position of the septum (III), the impedance gradually increases. At the central position of the septum (III), the impedance is maximal. As the ablation electrode 21 continues deeper towards the position away from the entering position from the central position of the septum (IV-V), its impedance gradually decreases. Therefore, it is possible to achieve identification of the position simply by monitoring changes in impedance of the ablation electrode 21 as the ablation catheter 10 moves within the body.

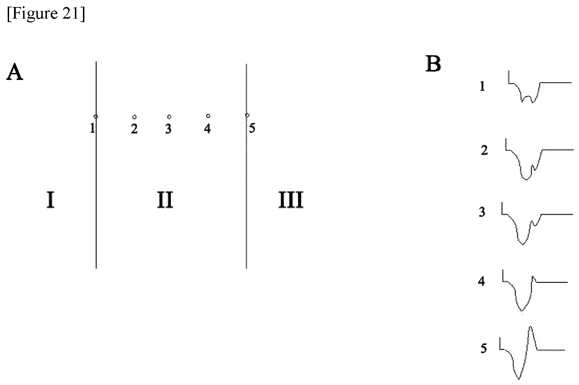

In addition, with an electrical stimulation/pacing signal emitted by the ablation electrode 21 in conjunction with the non-ablation electrode 31, the position of the ablation electrode 21 may also be determined by whether the above signal can be detected on the body surface and the difference in the signal. In brief, during entry of the ablation catheter 10, the ablation electrode 21 continuously emits an electrical stimulation/pacing signal which is not transmitted to the body surface and cannot be detected when the ablation electrode 21 does not enter into the target issue. However, once the ablation electrode 21 makes contact with the target issue, the signal is transmitted to the body surface and detected, resulting in changes observable on an electrocardiogram. Furthermore, differences in the emitted electrical signal occur when the target tissue is at either end of the interventricular septum. In particular, due to the presence of left and right bundle branches in the interventricular septum, the distance between these brunches and the signal source results in variations in the electrocardiogram when these brunches receive the electrical signal and feed back to the electrocardiogram,, enabling to determine the entering distance via changes in the electrocardiogram. When the ablation electrode 21 is at different positions (1-5) as shown in FIG. 21A, the electrocardiogram in lead V1 is illustrated in FIG. 21B, where I represents the right ventricle, II represents the interventricular septum, and III represents the left ventricle.

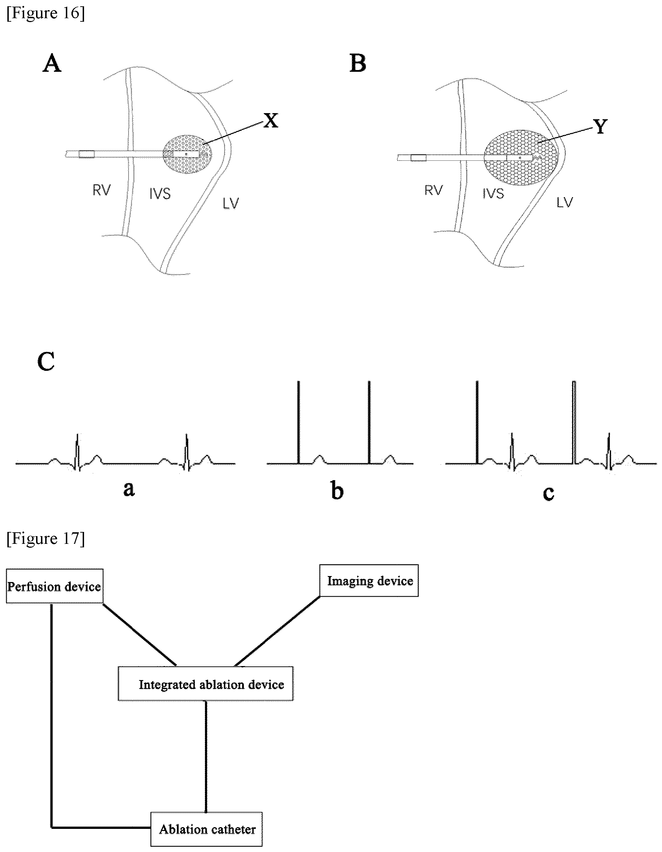

Furthermore, the ablation electrode 21 may also determine, after emitting ablation energy, the damage range of the target tissue by emitting the electrical stimulation/pacing signal in conjunction with the non-ablation electrode 31. In particular, the patient's own electrocardiogram is shown in FIG. 16C(a). According to the basic principle of ablation, cells in the target tissue undergo necrosis during radiofrequency ablation or apoptosis for irreversible electroporation during pulse ablation after completing ablation of the target tissue. Regardless of the type of ablation, within the damage range of the target tissue, the voltage below a predetermined value cannot be transmitted through the ablated site to the external undamaged tissue, and the electrocardiogram in this case is shown in FIG. 16C(c) as a post-ablation non-captured electrocardiogram. By applying an electrical signal (electrical stimulation/pacing signal) of gradually increasing voltage to the ablated tissue to mimic pacing functionality, stimulation is applied to the target tissue. Depending on whether the captured electrocardiogram can be detected on the body surface at different voltage values, the corresponding effective ablation range can be determined. When the applied pacing voltage signal is greater than or equal to a pacing voltage threshold, a captured electrocardiogram (as shown in FIG. 16C(b)) can be detected on the body surface, indicating that the damage range of the completed ablation is equivalent to a desired ablation damage range corresponding to the pacing voltage threshold. The pacing voltage threshold is a minimum voltage that can be transmitted through the ablated tissue to the non-ablated tissue after completing the desired ablation damage range. For example, assuming an ablation range of 10 mm (distance from the ablation catheter) and a corresponding pacing voltage threshold of 5V, if the applied voltage value is less than 5V, the target tissue will not respond, and the signal cannot be detected externally. However, if it exceeds 5V, the signal can be detected, allowing for determination of the range for this ablation. The pacing voltage threshold is directly proportional to the desired ablation damage range. As shown in FIGS. 16A and B, if ablation damage range X is less than ablation damage range Y, the pacing voltage threshold corresponding to the ablation damage range X will be less than that corresponding to the ablation damage range Y.

The perfusion hole 213 of the present invention is provided on the circumferential surface of the ablation electrode 21 and is capable of directly perfusing fluid into the target tissue. The fluid circulation mode is an external circulation mode, which offers superior cooling effects compared to the mode where the fluid circulates only within the ablation catheter 10. This is because the fluid is able to cool not only the ablation electrode 21 but also the target tissue, which enhances cooling efficiency. On the other hand, the cooling effect of internal circulation on the ablation electrode 21 is worse than that of external circulation at the same flow rate. Additionally, the second cavity 212 in the present invention can act as a buffer for the delivered fluid, reducing the pressure of the fluid flowing out of the perfusion hole 213 and preventing damage to the tissue caused by excessive pressure.

In one embodiment of the present invention, the perfusion hole 213 is provided on one side near the first cavity 211 of the second cavity 212, thereby enabling the second cavity 212 to function as a reservoir and reducing the pressure at the perfusion hole 213. As illustrated in FIG. 7, in one preferred embodiment, the perfusion hole 213 is set in a plurality of groups, and the plurality of groups of perfusion holes 213 are arranged spaced on the outer circumference surface of the second cavity 212 along the axial direction of the ablation electrode 21. In one preferred embodiment, each group of perfusion holes 213 includes a plurality of perfusion sub-holes arranged spaced circumferentially around the ablation electrode 21 (7B). In another preferred embodiment, the projection of two adjacent perfusion holes 213 overlaps partially on the plane perpendicular to the axial direction of the ablation electrode 21 (7A). Such arrangement allows for more uniform dispersion of the fluid, increased contact area with the ablation electrode 21 and the target tissue, and enhanced cooling efficiency. The shape of the perfusion hole 213 is not particularly limited and may be selected from one or more of circular, elliptical, cambered, fanned, arched, and polygonal shapes.

The structure of the connecting member 22 and the connection of the ablation body 2 and the catheter body 3 will be described with reference to FIGS. 8-11. FIG. 8 is a schematic sectional view of the connecting member 22 along a plane parallel to the X-axis, FIG. 9 is a schematic diagram showing connection of the connecting member 22 and the ablation electrode 21, FIG. 10 is a schematic sectional view of the connected connecting member 22 and ablation electrode 21 along a plane perpendicular to the X-axis, and FIG. 11 is a schematic sectional view of the connected ablation body 2 and catheter body 3 along a plane parallel to the X-axis. As shown in the figures, the connecting member 22 includes a first connecting portion 221 connected to the ablation electrode 21, a second connecting portion 222 connected to the catheter body 3, a first channel 223 axially extending through the connecting member 22, and a second channel 224 formed by the connecting member 22 and the inner wall of the first cavity 211 of the ablation electrode 21. The first connecting portion 221 is disposed within the first cavity 211, and the proximal end of the second connecting portion 222 extends from the ablation electrode 21 to connect with the catheter body 3. In one embodiment of the present invention, as shown in FIGS. 9 and 11, the outer diameter of the first connecting portion 221 of the connecting member 22 is slightly smaller than the inner diameter of the first cavity 211, such that the first connecting portion 221 can enter the first cavity 211. The portion near the second cavity 212 of the first cavity 211 has a flange 2111, and the first connecting portion 221 of the connecting member 22 abuts against the flange 2111. The second connecting portion 222 is a limit protrusion, and the catheter body 3 has a limit recess 35 near one side of the ablation body 2, which fits the second connecting portion 222. The connecting member is fixed to the catheter body 3 by inserting the second connecting portion 222 into the limit recess 35. The connecting member 22 may be fixedly connected with the ablation body 2 and the catheter body 3 by ways of bonding, riveting, press-fitting, or welding, or may be removably connected through snap-fitting or threaded connection, etc. The second channel 224 is used to configure other components such as the temperature sensor 23 and an electrical conductor wire 24 connected to the ablation electrode 21. In addition, those skilled in the art may add as needed the number of the second channel 224 and provide different components in different second channels 224, such as a positioning sensor, an electrical conductor wire joined with the screw-in member 11, and an electrical conductor wire joined with the insert 12, etc. The second channel 224 is not in communication with the second cavity 212, so that the fluid does not enter the second channel 224, thereby reducing interference of the fluid with components disposed in the second channel 224. Furthermore, disposing different components separately also reduces mutual interference between the components. The connecting member 22 is insulated, made of insulating material, or at least the surface is made of insulating material.

In addition, in some embodiments of the present invention, the ablation body 2 further comprises a positioning sensor located in one second channel 224. The positioning sensor may be located by electric field positioning, magnetic field positioning, electromagnetic field positioning, etc. In one embodiment of the present invention, the ablation catheter 10 determines the position of the ablation electrode 21 by a positioning sensor using magnetoelectric positioning, namely providing a magnetic field generator under the surgical area/bed to construct an overall magnetic field, wherein a positioning sensor with a magnetic coil is provided in the catheter, determining the position of the catheter by monitoring the change in magnetic field. In one embodiment, the positioning sensor of the present invention does not sense its position with respect to the target tissue. When two ablation catheters 10 of the present invention are simultaneously used for ablation, the positioning sensors each located on the two ablation catheter 10 sense the position of each other, and if ablation is performed when the distance therebetween reaches a predetermined distance threshold, the ablation effectiveness can be ensured to achieve complete ablation of the entire hypertrophic septum. The above distance threshold refers to, when the ablation damage ranges of two ablation catheters 10 are A and B, respectively, the maximum distance between the two ablative catheters 10 that allows A and B to at least partially overlap. The ablation damage range is related to the ablation energy released by the ablation electrode 21. Those skilled in the art can adjust and control an ablation parameter as needed to obtain the desired ablation damage range. For example, when the ablation energy is pulse, parameters such as pulse width, delay between pulses, pulse voltage may be adjusted. In one embodiment, two positioning sensors utilize microwave positioning to sense the distance between each other.

As shown in FIGS. 1 and 2, in some embodiments, the catheter body 3 comprises the non-ablation electrode 31. The non-ablation electrode 31 may be fabricated from any material commonly used in the art for electrode manufacturing without specific limitation, such as gold, copper, titanium alloy, platinum, stainless steel, etc. In one embodiment of the present invention, the non-ablation electrode 31 is a ring structure with a length of 0.5-5 mm. An electrical conductor wire is provided in the catheter body 3 to join with the non-ablation electrode 31 in order to achieve electrical connection with the control handle 4.

The non-ablation electrode 31 is not used to emit ablation energy, but to detect signal differences (such as impedance differences) from the ablation electrode 21 during the ablation process to determine the position of the ablation electrode 21, and to cooperate with the ablation electrode 21 to apply a pacing voltage electrical signal or to detect an electrocardiogram signal for determining the position of the ablation electrode 21 and the ablation damage range, etc. The non-ablation electrode 31 is spaced 2-30 mm (spacing D in FIGS. 1 and 2) from the ablation electrode 21. Within this distance, it is able to obtain an appropriate signal difference value more conveniently without affecting the ablation effectiveness. During the position determination which is similar to the distance determination using the ablation electrode 21 alone, due to different conductivities in blood, muscle, as well as in different regions of the same tissue, impedance between the ablation electrode 21 and the non-ablation electrode 31 may vary in different mediums. The impedance difference between the two varies with the position of the ablation catheter 10 in the vicinity of the target tissue, allowing for determination of the position of the ablation electrode 21 based on the impedance difference before ablation. In particular, upon entering the left and right ventricles without into the interventricular septum, the ablation electrode 21 and the non-ablation electrode 31 are both in contact with the blood within the ventricles, and their detected impedances are relatively close or almost identical. As the introducer portion 1 advances further, the ablation electrode 21 gradually contacts the target tissue (hypertrophic part of interventricular septum), while the spaced non-ablation electrode 31 remains in contact with the blood, resulting in differing impedances between the two electrodes. Furthermore, as the depth of the ablation electrode 21 changes (from partial contact to complete entry into the interventricular septum), the impedance difference increases gradually. Therefore, the degree of penetration can be determined by changes in impedance. Compared to using the ablation electrode 21 alone, using the ablation electrode 21 in conjunction with the non-ablation electrode 31 to determine the position through impedance differences provides a larger impedance difference value and thus a greater accuracy. The non-ablation electrode 31 may be plural and the spacing between a plurality of non-ablation electrodes 31 may be set at will.

Regarding the components that requires electrical conduction, such as the ablation electrode 21, the non-ablation electrode 31, the temperature sensor 23, the positioning sensor, etc., in the ablation catheter 10 of the present invention, it will be appreciated by those skilled in the art that a suitable conductor wire may be provided to join with each of them to achieve signal/electrical connection with the control handle 4.

In some embodiments, the catheter body 3 comprises an infusion channel 32, an elastic tube 33, and an outer tube 34, wherein the elastic tube 33 sleeves on the inner circumferential surface of the outer tube 34. In particular, as shown in FIG. 11, the elastic tube 33 is formed by winding a plurality of filaments around the inner circumferential surface of the outer tube 34. The filaments may be metallic materials or non-metallic materials with good elasticity. Since the ablation electrode 21 needs to be screwed into the target tissue for ablation, providing the elastic tube 33 enhances the overall support of the ablation catheter 10 and improves its resistance to bending during the twisting process. The elastic tube 33 is hollow to form the infusion channel 32 in communication with the ablation electrode 21 for delivering fluid.

In some embodiments, the catheter body 3 further comprises an adjustable bending portion located at one side near the ablation body 2 of the catheter body 3. The adjustable bending portion allows changing the angle of the distal end of the catheter body 3 and thus the position angle of the introducer portion 1 and the ablation body 2, facilitating surgical manipulation.

During ablation surgery, a liner wire enters the ablation catheter 10 from the proximal end of the ablation catheter 10 to guide and support the ablation catheter 10. Due to the small diameter of the guide wire, the diameter of the corresponding liner wire channel in the ablation catheter 10 is also small. Therefore, when inserting the liner wire into the liner wire channel, it is often difficult to align the liner wire with the entrance of the liner wire channel, causing a problem of hard insertion of the liner wire into the liner wire channel. Therefore, in some embodiments, the ablation catheter 10 of the present invention further includes a guiding member 6 for guiding the liner wire into the ablation catheter 10. As shown in FIGS. 12-13, the guiding member 6 comprises a connecting portion 61, a gripping portion 62, a guiding portion 63 which are provided in sequence from the distal end to the proximal end, and a guiding hole 64 extending through the connecting portion 61, the gripping portion 62 and the guiding portion 63. The connecting portion 61 is used to connect the guiding member 6 onto the ablation catheter 10. In one preferred embodiment, the connecting portion 61 is connected onto the ablation catheter 10 through threaded connection and includes a thread structure 611 corresponding to a thread structure on the ablation catheter 10 and a boss 612 that can enter into a groove correspondingly provided on the ablation catheter 10. In addition, the connecting portion 61 may be connected onto the ablation catheter 10 using other known methods in the art, such as snap-fitting or welding. The gripping portion 62 is located between the connecting portion 61 and the guiding portion 63, facilitating gripping and operation of a user. In some embodiments, the guiding member 6 may not include the gripping portion 62 and may be gripped through the connecting portion 61 and/or the guiding portion 63 during use. The inner wall of the guiding portion 63 is in a trumpet shape which tapers from one end away from the connecting portion 61 to one end near the connecting portion 61. The opening near the user in the guiding portion 63 is larger, facilitating the entry of the small-diameter liner wire into the guiding portion 63. As the diameter of the opening deceases gradually, even if the liner wire initially does not directly enter the guiding hole 64, it can slide into the guiding hole 64 owing to the tapered inner wall of the guiding portion 63, making the operation simple and saving operation time. The guiding hole 64 extends axially through the guiding member 6 and communicates with the liner wire channel in the ablation catheter 10, allowing the liner wire entering the guiding member 6 to enter the ablation catheter 10 directly. In one embodiment of the present invention, the liner wire channel and the infusion channel 32 are the same channel.

In one embodiment of the present invention, as shown in FIG. 14, the ablation catheter 10 comprises a three-way valve 5 joined with the control handle 4. One opening of the three-way valve 5 may be in communication with a perfusion device, and the guiding member 6 is joined with another opening of the three-way valve 5. The other end of the guiding member 6 is joined with a torque transmission member 7, with the liner wire fixed in the center hole of the torque transmission member 7. Because the liner wire is thin, a user may feel difficult to grip and manipulate it. The torque transmission member 7 is provided to allow manipulation of the liner wire by operating the torque transmission member 7, transmitting the torque generated by twisting at the proximal end to the distal end.

FIG. 17 is a schematic diagram of an ablation system according to the present invention. As shown in FIGS. 17-18, the ablation system of the present invention comprises an integrated ablation device, and the ablation catheter 10 of the present invention, a perfusion device, and an imaging system connected to the integrated ablation device. The perfusion system is also joined with the ablation catheter 10 for supplying fluid for the ablation catheter 10 under the control of the integrated ablation device. In some embodiment, the perfusion fluid is saline. In other embodiments, the perfusion device may accept control from the integrated ablation device to perfuse other fluids into the ablation catheter 10, as long as it can increase the electrical and thermal conductivity of the ablation tissue, maintain impedance balance, keep impedance relatively stable, reduce the temperature of the ablation tissue, increase the humidity of the ablation tissue, and fundamentally prevent scabbing due to drying and heating of the ablation tissue without causing serious side effects to the human body. The perfusion rate of the perfusion device is adjustable, and during perfusion, the rate can be controlled in real-time by the integrated ablation device, with an output capacity of at least 0.05-10.0 mL/min. The imaging device is used to display an image of the ablation catheter 10 in the human body during surgery and may be one or a combination of more of a 3D imaging system, CT, MR, DSA, etc. In the present invention, to reduce surgical requirements and hospital costs, and expand the use of the interventricular septal ablation system, the imaging device in this system is a DSA device that meets minimal surgical imaging requirements, avoiding the need for a complete and expensive set of imaging system, thereby allowing non-tier 3A township hospitals to meet surgical conditions, expanding the benefits of surgery and facilitating promotion. Compared to CT, DSA can also reduce radiation doses to an operator and increase safety. When pulse ablation is performed, a negative plate 20 is required outside the patient's body.

The integrated ablation device comprises an ablation generation module, a temperature detection module, an impedance detection module, an ECG signal detection module, an electrical stimulation/pacing signal module, a MCU central control module, an interactive control module, and an ablation control switch. Among them, the ablation generation module is used to generate and deliver ablation energy to the ablation electrode 21, which may be radio frequency energy or pulse energy. In addition, in one preferred embodiment, the ablation generation module can also provide one or more of low-temperature energy, laser, chemical reagents, electroporation, high-intensity focused ultrasound or ultrasound, and microwave energy. The temperature detection module is used to receive a signal from the temperature sensor 23 of the ablation catheter 10 and feed back to the MCU central control module The impedance detection module is used to detect the impedance of the ablation electrode 21 and/or the non-ablation electrode 31 and feed back to the MCU central control module. The ECG signal detection module is used to detect an electrocardiogram signal and feed back to the MCU central control module. The electrical stimulation/pacing signal module is used to generate and send an electrical stimulation/pacing signal through the ablation electrode 21 under the control of the MCU central control module. The MCU central control module is in signal connection with the ablation generation module, the temperature detection module, the impedance detection module, the ECG signal detection module, the electrical stimulation/pacing signal module, the interactive control module, the ablation control switch, the ablation catheter 10, the perfusion device, and the imaging device for receiving information from other modules for processing and providing feedback and/or display of processed information. For example, the MCU central control module controls the perfusion device to change the perfusion rate based on a customized threshold temperature after receiving a temperature fed back by the temperature detection module. When the temperature exceeds the threshold temperature, the perfusion rate increases, and when it falls below the threshold temperature, the perfusion rate decreases. Preferably, the customized threshold temperature ranges between 40 to 70 degrees Celsius, preferably 55 degrees Celsius. Except in the stopped state, there is a requirement to set a minimum flow rate threshold for the perfusion rate during perfusion maintained, preferably at 0.5 mL/min. The interactive control module is used to display information and receive a control instruction from a user. In one embodiment, the interactive control module may be a touch screen. The ablation control switch is used to control application of ablation energy to the target tissue. In one embodiment, the ablation control switch is a foot switch through which ablation function begins when the user pedals, and a predetermined time may be customized in the meanwhile, such as 3 seconds of continuous pedaling to control different ablation functions/modes. In one embodiment of the present invention, the ablation system further includes a position detection module. When the ablation catheter 10 includes a positioning sensor, the position detection module receives a signal from the positioning sensor and provides feedback to the MCU central control module.

When using the ablation system described above for ablation, the operation is performed according to the following steps: S1: Subclavian vein/jugular vein puncture, sending the ablation catheter 10 into the right ventricle via a delivery catheter; S2: Under the guidance of the imaging device, adjusting the angle and position of the delivery catheter and the ablation catheter 10, and monitoring impedance and/or electrocardiogram waveforms in real time, wherein the impedance is impedance between the ablation electrode 21 and the negative plate 20 on the patient's body surface and/or impedance difference between the ablation electrode 21 and the non-ablation electrode 31; S3: After the delivery catheter reaches the designated position, screwing the introducer portion 1 into the target tissue, determining, during the process, whether the introducer portion 1 is screwed in and the depth of screwing in based on changes in impedance between the ablation electrode 21 and the negative plate 20 of the patient's body surface and/or changes in impedance difference between the ablation electrode 21 and the non-ablation electrode 31, and activating the perfusion device once the ablation electrode 21 reaches a target ablation area; S4: Adjusting a parameter for ablation; S5: After completing ablation, performing electrical stimulation to check if the ablation is sufficient; S6: Unscrewing; S7: Repeating steps S2-S6 if ablation of additional sites are required; S8: After completing all operations, removing the catheter from the body.

In one preferred embodiment of the present invention, the ablation catheter 10 includes a positioning sensor, and step S2 further includes that the ablation catheter 10 determines the position of the ablation electrode 21 by the positioning sensor using magnetoelectric positioning. In one embodiment, different from subclavian vein/jugular vein puncture and sending the ablation catheter 10 into the right ventricle via a delivery catheter as described above, alternatively, in step S1, femoral artery is punctured, sending the ablation catheter 10 into the left ventricle via the delivery catheter. In one embodiment, in step S3, an electrical signal may be emitted by the ablation electrode 21, and whether the introducer portion 1 is screwed in and the depth of screwing in is determined based on whether the electrical signal can be detected on the body surface as well as a change in the electrical signal. In one embodiment, in step S5, the electrical stimulation check involves applying a progressively increasing pacing voltage electrical signal to the ablated tissue via the ablation electrode 21. When the applied pacing voltage electrical signal exceeds or equals a pacing voltage threshold, and an electrocardiogram captured by the electrical signal can be detected on the body surface, the completed ablation damage range is a desired ablation range corresponding to the pacing voltage threshold. The pacing voltage threshold represents a minimum voltage that, after completing the desired ablation damage range, can transmit to non-ablated tissue via ablated tissue, wherein the pacing voltage threshold is proportional to the desired ablation damage range.

In one embodiment, the introducer portion 1 is electrically insulated, and the ablation electrode 21 emits radio frequency energy to the target tissue, causing thermal damage to cells in the target tissue and thus achieving ablation. In another embodiment, the introducer portion 1 is electrically insulated, and the ablation electrode 21 emits pulse energy to the target tissue. In this case, the negative plate 20 is provided on the body surface, causing irreversible cell electroporation in the target tissue, thereby achieving ablation. In another embodiment, the introducer portion 1 is conductive and opposite in polarity to the ablation electrode 21. The ablation electrode 21 emits pulse energy to the target tissue. At this time, the ablation catheter 10 functions as a pulse bipolar ablation catheter, causing irreversible cell electroporation in the target tissue and achieving ablation. In another embodiment, the introducer portion 1 is electrically insulated, and the ablation body includes at least two ablation electrodes 21 with opposite polarities. The ablation electrode 21 emits pulse energy to the target tissue. At this time, the ablation catheter 10 functions as a pulse bipolar ablation catheter, causing irreversible cell electroporation in the target tissue, and thereby achieving ablation.

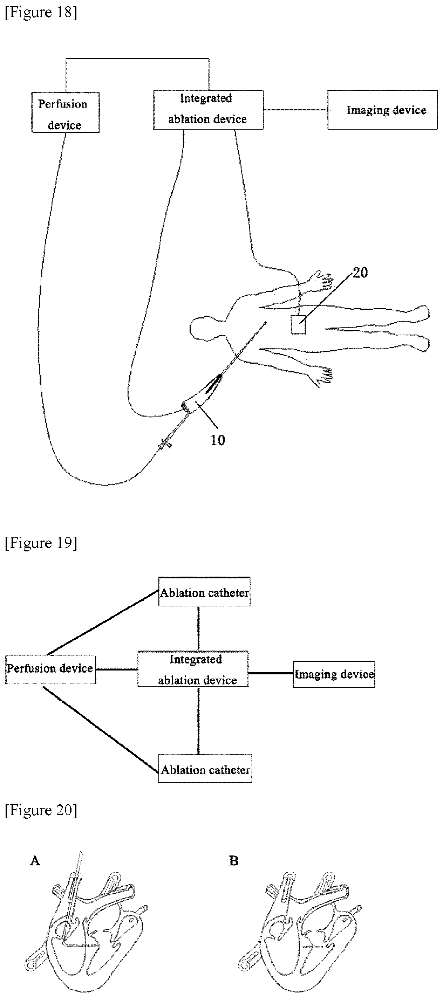

FIG. 19 illustrates a schematic diagram of another ablation system of the present invention. In contrast to the ablation system with a single ablation catheter as shown in FIG. 17, the ablation system in this embodiment includes two ablation catheters 10, which are each connected to the integrated ablation device and the perfusion device, with other components identical to the ablation system as shown in FIG. 17. FIG. 20 shows the situation where a single ablation catheter is used for ablation in the left ventricle (A) and right ventricle (B). When using the ablation system of this embodiment, subclavian vein/jugular vein puncture is performed, sending one of the ablation catheters 10 into the right ventricle via a delivery catheter and screwing from the right ventricle into the interventricular septum. The other ablation catheter 10 is sent into the left ventricle via the delivery catheter through femoral artery puncture, screwing from the left ventricle into the interventricular septum. Other steps are the same as the operating steps of the ablation system with a single ablation catheter shown in FIG. 17.

Depending on actual requirements, the ablation system of the present invention may comprise more than two ablation catheters 10, which are each connected to the integrated ablation device and the perfusion device respectively. The connection method and operation method of other components are the same as those in the ablation system shown in FIGS. 17 and 19, and those skilled in the art can operate more than two ablation catheters 10 based on the above description of the present invention.

In addition, regarding the polarity of the ablation electrode 21 and whether the introducer portion 1 is conductive, the ablation system shown in FIG. 19 can also be arranged as follows: both introducer portions 1 of the two ablation catheters 10 are electrically insulated, the two ablation electrodes 21 have the same/different polarity but are unrelated to each other, emitting pulse energy with the negative plate 20 attached to the body surface, and each ablation electrode 21 performs single-stage ablation; or both introducer portions 1 of the two ablation catheters 10 are electrically insulated, and the two ablation electrodes 21 have opposite polarity, emitting pulse energy, working together during ablation and achieving bipolar ablation, which can effectively increase the ablation area.

Although medical judgment for hypertrophic cardiomyopathy is that the interventricular septum thickness exceeds 15 mm, in practice, a patient is often admitted with a thickness of more than 20 mm, with some reaching 30 mm. For a patient with such thickness, it is necessary to deepen the screwing in. However, on one hand, after screwing in, torque transmission may be hindered by myocardial tissue, and deeper screwing in becomes increasingly difficult. On the other hand, the size of the ablation range is limited, and excessive depth implies incomplete ablation. Therefore, by sending at least two ablation catheters 10 into the left/right ventricle for simultaneous ablation, the ablation area can be effectively expanded, and the difficulty of screwing in is reduced.

Additionally, although the safety of pulse ablation is improved compared to radio frequency ablation, the size of the ablation area is limited. In a case where the device remains unchanged, in order to enlarge the ablation area, pulse width/voltage needs be increased. However, such increase, when exceeding a threshold, may possibly result in physiological responses in a patient, such as muscle twitch. The ablation system shown in FIG. 19 provides at least two ablation catheters 10, such that ablation for thickened interventricular septum due to hypertrophic cardiomyopathy can be performed simultaneously from both sides without increasing pulse width/voltage, achieving superior ablation effectiveness while enhancing safety.

In one preferred embodiment of the present invention, at least one of the two ablation catheters 10 includes a positioning sensor. The positioning sensor is configured to use magnetoelectric positioning to determine the position of the ablation catheter 10 comprising the positioning sensor and transmit the position signal to the integrated ablation device. Additionally, in one preferred embodiment, the two ablation catheters 10 each includes a positioning sensor. The two positioning sensors are configured to use microwave positioning to sense the distance between each other and transmit the distance signal to the integrated ablation device. The advantage of this solution is that, compared to rough judgment of entering distance and position based on impedance and electrocardiogram waveforms, judgment by the position signal transmitted through positioning sensors, especially the distance between the two positioning sensors, is more accurate and easier to implement without the need for cooperation with any other expensive devices. For hospitals, existing determination of position by ablation catheters must be accompanied by a complete three-dimensional mapping system, which is priced in the millions and is an expense that ordinary hospitals find difficult to bear, limiting the development of an ablation surgery. Through the easily implemented positioning sensor, it is possible to complete screwing in and determine the position under the guidance of the simplest imaging device, significantly reducing the basic requirements for conducting a surgery and lowering the learning difficulty for a doctor.

In conclusion, the present invention provides an ablation catheter, an ablation system comprising the ablation catheter and an ablation method. By fixing the introducer portion within the target tissue, the ablation position is easily fixed and remains unchanged during operation. And, setting of the non-ablation electrode enables detection of impedance, pacing voltage, etc., and facilitates detection of the position of the ablation catheter and its screw-in depth, etc., without requiring an expensive three-dimensional imaging device. Furthermore, when using the ablation system comprising at least two ablation catheters in the present invention, it may be bipolar ablation which is more effective. Bi-directional screwing in of the two ablation catheters prevents excessive screwing in of a single catheter, thereby reducing tissue damage, expanding the ablation area. Additionally, the precise position of the ablation catheter can be detected at a low cost through a positioning sensor.

The embodiments of the present invention have been described in detail with reference to the accompanying drawings. However, the present invention is not limited to the embodiments described above. Various components of the present invention may be used in any combinations. Within the knowledge of those skilled in the art, various modifications may be made without departing from the spirit of the present invention.

## Claims

1. An ablation catheter comprising an introducer portion, an ablation body, a catheter body and a control handle which are connected in sequence;
wherein:
the introducer portion comprises a screw-in member capable of being rotated into and fixed to the target tissue under external force;
the ablation body comprises an ablation electrode, a connecting member and a temperature sensor; the ablation electrode has a first end connected with the screw-in member, a first cavity arranged opposite the first end, a second cavity formed by extending from the first cavity towards the first end and a perfusion hole extending through a circumferential surface of the ablation electrode; inside the first cavity is provided the connecting member, and one end of the connecting member, which is near the catheter body, extends beyond the first cavity and joins with the catheter body; the connecting member has a first channel running through axially, the first channel communicates with the second cavity, the perfusion hole, and an infusion channel in the catheter body; the connecting member and an inner wall of the first cavity form a number of second channels, with the temperature sensor located in one of the second channels;
the catheter body comprises a non-ablation electrode and the infusion channel, with the non-ablation electrodes spaced from the ablation electrode, and the non-ablation electrode is not in contact with a target tissue during ablation.

2. The ablation catheter of claim 1, wherein position information for the ablation electrode is provided by the impedance difference between the ablation electrode and the non-ablation electrode.

3. The ablation catheter of claim 1, wherein the screw-in member is in a three-dimensional helical line shape or screw shape, with an end away from the ablation body as a tip.

4. The ablation catheter of claim 3, wherein the pitch of the screw-in member gradually decreases from a distal end to a proximal end, and the pitch of the screw-in member is 0.25-1.5 times the diameter of the screw-in member.

5. The ablation catheter of claim 3, wherein the diameter of the screw-in member gradually decreases from a proximal end to a distal end.

6. The ablation catheter of claim 1, wherein the first end of an ablation electrode has a third cavity into which the screw-in member is connected.

7. The ablation catheter of claim 6, wherein the screw-in member is fixedly connected into the third cavity, and the length of the screw-in member is greater than the length of the third cavity in an axial direction.

8. The ablation catheter of claim 6, wherein the screw-in member is telescopically connected into the third cavity, and the length of the screw-in member is less than or equal to the length of the third cavity in an axial direction.

9. The ablation catheter of claim 1, wherein the introducer portion further includes an insert, the length of which is less than or equal to the length of the screw-in member, and the insert is sleeved inside the screw-in member and does not contact the screw-in member.

10. The ablation catheter of claim 9, wherein the insert has a hollow structure and a through hole extending through the circumferential surface of the insert, and a proximal end of the insert is in communication with the second cavity.

11. The ablation catheter of claim 1, wherein the perfusion hole is provided on one side near the first cavity of the second cavity.

12. The ablation catheter of claim 1, wherein the perfusion hole is set in a plurality of groups, and the plurality of groups of perfusion holes are arranged spaced on an outer circumference surface of the second cavity along an axial direction of the ablation electrode.

13. The ablation catheter of claim 12, wherein each group of the perfusion holes comprises a plurality of perfusion sub-holes which are spaced circumferentially around the ablation electrode.

14. The ablation catheter of claim 12, wherein the projection of two adjacent perfusion holes overlaps partially on the plane perpendicular to the axial direction of the ablation electrode.

15. The ablation catheter of claim 1, wherein the introducer portion conducts electricity and is insulated from the ablation electrode.

16. The ablation catheter of claim 1, wherein the ablation body further comprises a positioning sensor configured to sense the position of the ablation catheter in a target tissue, and the temperature sensor and the positioning sensor are located in different second channels.

17. The ablation catheter of any one of claims 1-16, wherein the ablation catheter further comprises a guiding member comprising a connecting portion, a guiding portion, and a guiding hole extending through the connecting portion and the guiding portion; the connecting portion is joined with the ablation catheter; the inner wall of the guiding portion is in a trumpet shape which tapers from one end away from the connecting portion to one end near the connecting portion; the guiding hole is in communication with a liner wire channel in the ablation catheter.

18. The ablation catheter of claim 17, wherein the guiding member further comprises a gripping portion located between the connecting portion and the guiding portion, and the guiding hole extends through the connecting portion, the gripping portion and the guiding portion.

19. An ablation system comprising the ablation catheter of any one of claims 1-18, an integrated ablation device, a perfusion device, and an imaging device; the integrated ablation device is connected to the ablation catheter, the perfusion device and the imaging device respectively, and the perfusion device is joined with the ablation catheter; the integrated ablation device comprises an ablation generation module, a temperature detection module, an impedance detection module, an ECG signal detection module, an electrical stimulation/pacing signal module, a MCU central control module, an interactive control module and an ablation control switch;
wherein the ablation generation module is used to generate and deliver ablation energy to the ablation electrode;
the temperature detection module is used to receive a signal from the temperature sensor and feed back to the MCU central control module; the impedance detection module is used to detect the impedance of the ablation electrode and/or the non-ablation electrode and feed back to the MCU central control module;
the ECG signal detection module is used to detect an electrocardiogram signal and feed back to the MCU central control module;
the electrical stimulation/pacing signal module is used to generate and send an electrical stimulation/pacing signal through the ablation electrode under control of the MCU central control module;
the MCU central control module is in signal connection with the ablation generation module, the temperature detection module, the impedance detection module, the ECG signal detection module, the electrical stimulation/pacing signal module, the interactive control module, the ablation control switch, the ablation catheter, the perfusion device and the imaging device for receiving information from other modules for processing and providing feedback and/or display of processed information;
the interactive control module is used to display information and receive a control instruction from a user;
the ablation control switch is used to control application of ablation energy to a target tissue.

20. An ablation system, comprising at least two ablation catheters of any one of claims 1-18, an integrated ablation device, a perfusion device, and an imaging device; the integrated ablation device is connected to the at least two ablation catheters, the perfusion device and the imaging device respectively, and the perfusion device is joined with the at least two ablation catheters; the integrated ablation device comprises an ablation generation module, a temperature detection module, an impedance detection module, an ECG signal detection module, an electrical stimulation/pacing signal module, a MCU central control module, an interactive control module, and an ablation control switch;
wherein the ablation generation module is used to generate and deliver ablation energy to the ablation electrode;
the temperature detection module is used to receive a signal from the temperature sensor and feed back to the MCU central control module;
the impedance detection module is used to detect the impedance of the ablation electrode and/or the non-ablation electrode and feed back to the MCU central control module;
the ECG signal detection module is used to detect an electrocardiogram signal and feed back to the MCU central control module;
the electrical stimulation/pacing signal module is used to generate and send an electrical stimulation/pacing signal through the ablation electrode under control of the MCU central control module;
the MCU central control module is in signal connection with the ablation generation module, the temperature detection module, the impedance detection module, the ECG signal detection module, the electrical stimulation/pacing signal module, the interactive control module, the ablation control switch, the at least two ablation catheters, the perfusion device and the imaging device for receiving information from other modules for processing and providing feedback and/or display of processed information;
the interactive control module is used to display information and receive a control instruction from a user;
the ablation control switch is used to control application of ablation energy to a target tissue.

21. The ablation system of claim 19 or 20, wherein the ablation system further comprises a position detection module which receives a signal from a positioning sensor and feeds back to the MCU central control module.

22. An ablation method using the ablation system of claim 19, comprising the steps of:
S1: Subclavian vein/jugular vein puncture, sending the ablation catheter into the right ventricle via a delivery catheter;
S2: Under the guidance of an imaging device, adjusting the angle and position of the delivery catheter and the ablation catheter, and monitoring impedance and/or electrocardiogram waveforms in real-time, wherein the impedance is impedance between the ablation electrode and a negative plate on the patient's body surface, and/or impedance difference between the ablation electrode and the non-ablation electrode;
S3: After the delivery catheter reaches the designated position, screwing the introducer portion into a target tissue, determining, during the process, whether the introducer portion is screwed in and the depth of screwing in based on changes in impedance between the ablation electrode and the negative plate on the patient's body surface and/or changes in impedance difference between the ablation electrode and the non-ablation electrode, and activating the perfusion device once the ablation electrode reaches a target ablation area;
S4: Adjusting a parameter for ablation;
S5: After completing ablation, performing electrical stimulation to check if the ablation is sufficient;
S6: Unscrewing;
S7: Repeating S2-S6 if ablation of additional sites is required;
S8: After completing all operations, removing the catheter from the body.

23. The ablation method of claim 22, wherein the ablation catheter includes a positioning sensor, and step S2 further comprises that the ablation catheter determines the position of the ablation electrode by the positioning sensor using magnetoelectric positioning.

24. The ablation method of claim 22, wherein in step S1, femoral artery puncture is performed, sending the ablation catheter into the left ventricle via a delivery catheter.

25. The ablation method of any one of claims 22-24, wherein in step S3, an electrical signal is emitted by the ablation electrode, and whether the introducer portion is screwed in and the depth of screwing in are determined based on whether an electrical signal can be detected on the body surface and a change in the electrical signal.

26. The ablation method of any one of claims 22-24, wherein in step S5, the electrical stimulation check involves applying a progressively increasing pacing voltage electrical signal to the ablated tissue via the ablation electrode, and when the applied pacing voltage electrical signal exceeds or equals a pacing voltage threshold, and an electrocardiogram captured by the electrical signal can be detected on the body surface, the completed ablation damage range is a desired ablation range corresponding to the pacing voltage threshold; the pacing voltage threshold represents a minimum voltage that, after completing a desired ablation damage range, can be transmitted to non-ablated tissue via ablated tissue, and the pacing voltage threshold is directly proportional to a desired ablation damage range.
